# EUROPÄISCHE PATENTSCHRIFT

(11) **EP 3 132 765 B1**
(45) Veröffentlichungstag und Bekanntmachung des Hinweises auf die Patenterteilung: **30.03.2022**
(21) Anmeldenummer: 15182008.1
(22) Anmeldetag: 21.08.2015
(51) Int. Cl.: A61B 18/14

(54) **KOAGULATIONS- UND DISSEKTIONSINSTRUMENT MIT VERBESSERTER BEDIENUNG**
COAGULATION AND DISSECTION INSTRUMENT WITH IMPROVED CONTROL
INSTRUMENT DE COAGULATION ET DE DISSECTION A COMMANDE AMELIOREE

(43) Veröffentlichungstag der Anmeldung: 22.02.2017
(73) Patentinhaber: Erbe Elektromedizin GmbH, 72072 Tübingen (DE)
(72) Erfinder: Weiler, Rolf, 72127 Kusterdingen (DE); Fritz, Martin, 72070 Tübingen (DE); Schall, Heiko, 72622 Nürtingen (DE)
(74) Vertreter: Rüger Abel Patentanwälte PartGmbB

(56) Entgegenhaltungen:
- EP-A1- 2 792 326
- WO-A1-00/47124
- WO-A1-2015/093409
- US-A1- 2004 054 365
- US-A1- 2013 274 729
- US-A1- 2014 214 019
- US-A1- 2016 249 975

## Beschreibung

Die Erfindung betrifft ein Instrument zur Dissektion und Koagulation oder bedarfsweise nur zur Koagulation von biologischem Gewebe.

Instrumente zur Koagulation und Dissektion von biologischem Gewebe sind beispielsweise aus der US 8 394 094 B2 bekannt. Das Instrument ist nach Art einer Zange ausgebildet, zwischen deren Branchen gefasstes Gewebe mittels elektrischen Stroms koaguliert wird. Dazu sind an einer der Branchen sowohl Koagulationselektroden als auch eine Schneidelektrode angebracht. Die andere Branche dient als Gegenelektrode.

Ein ähnliches Instrument ist aus der WO 00/47124 bekannt. Das Instrument enthält eine Transformatoranordnung, die von einem elektrischen Generator gespeist wird. Die Transformatoranordnung erzeugt sowohl die Koagulationsspannung als auch die Schneidspannung. Damit können Koagulation und Dissektion gleichzeitig durchgeführt werden.

Ein weiteres zur gleichen Gattung gehöriges Instrument ist aus der EP 1 958 583 A2 bekannt, dessen Werkzeug wiederum Koagulationselektroden und mindestens eine Schneidelektrode enthält. Zur Aktivierung dient eine Schalteranordnung, die am Handgriff des Instruments angebracht ist und die Aktivierung der Elektroden gestattet.

Zur elektrischen Steuerung des Koagulations- und Dissektionsprozesses offenbart die US 2005/0171533 A1 ein Instrument mit Koagulations- und Schneidelektrode, die aus einem HF-Generator mit Leistung versorgt werden. Das Instrument enthält eine Steuerungseinrichtung, die zwei Schalter nacheinander betätigt, um zunächst die Koagulationselektroden und dann die Schneidelektrode zu aktivieren. Die Steuerung der Aktivierung wird durch Anzapfung von Transformatorwicklungen bewirkt.

Bei der praktischen Anwendung kann es wünschenswert sein, die Abläufe der Koagulation und Dissektion manuell zu steuern. Dabei kann es vorkommen, dass der Anwender zum Beispiel nur eine Koagulation oder nur eine Dissektion durchführen will oder dass er nach Koagulation und Dissektion nochmals koagulieren will.

Aus der WO 2015/093409 A1 sowie der US 2016/0249975 A1 ist ein medizinisches zangenartiges Instrument mit Elektroden bekannt von denen wenigstens eine in einen distalen und einen proximalen Abschnitt unterteilt ist. Ein Aktivierungsschalter ist dazu ausgelegt, wahlweise den distalen Abschnitt allein, den proximalen Abschnitt allein oder beide Abschnitte gemeinsam zu aktivieren, je nachdem wie weit der Schalter betätigt wird. Das Dokument US 2004/054365 A1 offenbart ebenfalls relevanten Stand der Technik.

Es ist Aufgabe der Erfindung, ein Instrument anzugeben, das eine verlässliche manuelle Steuerung des Koagulierens und Schneidens ermöglicht, wobei dazu eine technisch möglichst einfache Lösung angestrebt wird.

Diese Aufgabe wird mit dem Instrument nach Anspruch 1 gelöst:

Das erfindungsgemäße Instrument kann zur bedarfsweisen Koagulation und Dissektion von biologischem Gewebe eingesetzt werden. Es umfasst ein Werkzeug mit wenigstens zwei Koagulationselektroden, von denen mindestens eine in Bezug auf die andere beweglich ist, so dass sie insgesamt gesehen relativ zueinander beweglich sind. Sie dienen dazu, zwischen einander Gewebe zu fassen und, falls gewünscht, mechanisch zusammen zu drücken. Weiter ist wenigstens eine Schneidelektrode vorgesehen, um zwischen den Koagulationselektroden gefasstes Gewebe bedarfsweise zu durchtrennen. Die Trennung erfolgt dabei wiederum durch elektrische Einwirkung. Sie kann durch mechanische Einwirkung der Schneidelektrode auf das Gewebe unterstützt sein.

In dem Instrument ist eine elektrische Schaltung vorgesehen, die ausgangsseitig an die Koagulationselektroden und an die Schneidelektrode angeschlossen ist. Eingangsseitig ist die elektrische Schaltung an eine Versorgungsspannungsquelle anschließbar. Die Versorgungsspannungsquelle ist vorzugsweise eine HF-Ausgangsspannungsquelle. Die HF-Ausgangsspannungsquelle speist eine Schneidspannungsquelle sowie eine Koagulationsspannungsquelle, die Bestandteil der elektrischen Schaltung sein können. Es ist jedoch auch möglich, die Schneidspannungsquelle und die Koagulationsspannungsquelle extern bereit zu stellen, wobei das Instrument mit seiner elektrischen Schaltung an diese Quellen anschließbar ist. Die Schneidspannungsquelle ist durch einen Ausgang eines Transformators gebildet, der eingangsseitig an die HF-Ausgangsspannungsquelle angeschlossen ist. Der Transformator ist im Instrument angeordnet.

Die elektrische Schaltung enthält einen Leistungsschalter zur wahlweisen Trennung oder Zuschaltung der Schneidelektrode von bzw. zu der Schneidspannungsquelle sowie einen Schalter zur wahlweisen Aktivierung der Koagulationsspannungsquelle bzw. der HF-Ausgangsspannungsquelle. Ein in dem Instrument vorhandener Transformator kann als Koagulationsspannungsquelle dienen und zugleich von der Koagulationsspannung gespeist werden, die von der HF-Ausgangsspannungsquelle geliefert werden kann. Die Aktivierung der Koagulationsspannungsquelle kann direkt erfolgen, indem die Koagulationsspannungsquelle (z.B. der Transformator) als solche aktiviert (ein- und ausgeschaltet) wird. Bevorzugt erfolgt die Aktivierung indirekt, indem der Koagulationsaktivierungsschalter zur Aktivierung (und Deaktivierung) der HF-Ausgangsspannungsquelle dient, die beispielsweise durch einen HF-Generator eines speisenden medizinischen Geräts gebildet ist, an das das Instrument angeschlossen ist. Erhält das Instrument von der HF-Ausgangsspannungsquelle Versorgungsspannung, ist die Koagulationsspannungsquelle aktiv.

Bei dem erfindungsgemäßen Instrument sind der Leistungsschalter und der Steuerschalter zur Aktivierung der Koagulationsspannungsquelle von einem gemeinsamen Betätigungselement seriell gesteuert. Der Steuerschalter kann ein Koagulationsaktivierungsschalter oder ein Schneidaktivierungsschalter sein. Die serielle Steuerung des Steuerschalters und des Leistungsschalters bedeutet, dass eine manuelle Betätigung des Betätigungselements zunächst dazu führt, dass einer der Schalter, vorzugsweise der Leistungsschalter, betätigt, d.h. geöffnet oder geschlossen wird, und dass der Schalter zur Aktivierung der Koagulationsspannungsquelle erst danach, d.h. dann geschlossen wird, wenn der Leistungsschalter den zur Schneidelektrode führenden Strompfad bereits unterbrochen oder eben bereits geschlossen hat. Auf diese Weise ist beim Koagulieren die Schneidelektrode von der Schneidspannungsquelle getrennt, bevor eine Aktivierung, zum Beispiel der HF-Ausgangsspannungsquelle, erfolgt. Bei der alternativen Ausführungsform mit als Schließer ausgebildetem Leistungsschalter ist die Schneidelektrode mit der Schneidspannungsquelle verbunden, bevor eine Aktivierung erfolgt. Somit können die Koagulationsspannungsquelle und die Schneidspannungsquelle gleichzeitig aktiviert werden, ohne dass im Koagulationsmodus die Schneidelektrode Schneidspannung erhalten würde.

Mit diesem Konzept lässt sich mit einer besonders einfachen Schaltung ein sehr verlässlich arbeitendes feinfühlig zu steuerndes Instrument erstellen, das an einen HF-Generator mit zweipoligem Ausgang anschließbar und wahlweise sowohl zum Koagulieren als auch zum Schneiden verwendbar ist, wobei das Umschalten mit einfachsten Mitteln direkt am Instrument manuell erfolgen kann.

Bei dem erfindungsgemäßen Instrument sind der Leistungsschalter und der Koagulationsaktivierungsschalter derart mit dem Betätigungselement verbunden, dass bei einer Betätigung des Betätigungselements zunächst der Leistungsschalter öffnet, bevor der Koagulationsaktivierungsschalter schließt, und dass bei einer Freigabe des Betätigungselements der Koagulationsaktivierungsschalter öffnet, bevor der Leistungsschalter schließt. Dies stellt sicher, dass im reinen Koagulationsmodus stets die Schneidelektrode von der Schneidspannungsquelle getrennt ist, und zwar unabhängig davon, ob diese tatsächlich aktiv ist oder nicht. Mit dem genannten Konzept wird die Möglichkeit eröffnet, die Schneidspannungsquelle an einem Schaltungspunkt auszubilden bzw. bereitzustellen, der transformatorisch fest an die Koagulationsspannung gekoppelt ist, wobei die Koagulationsspannung von der HF-Ausgangsspannungsquelle geliefert wird. Dies führt zu einem einfachen Aufbau des Instruments.

Der Leistungsschalter, der den Schneidstrom führt, d.h. die Schneidspannungsquelle mit der Schneidelektrode verbindet, ist vorzugsweise ein Ruhekontakt. Das heißt er stellt in nicht betätigtem Zustand (Ruhezustand) eine elektrische Verbindung zwischen der Schneidspannungsquelle und der Schneidelektrode her. In betätigtem Zustand trennt er hingegen die Schneidelektrode von der Schneidspannungsquelle. In betätigtem Zustand nimmt er den Koagulationsmode ein. In diesem kann er die Schneidelektrode mit der Koagulationsspanungsquelle verbinden. In diesem Fall kann die Schneidelektrode zur Koagulationswirkung beitragen. Es kann jedoch auch gewünscht sein, die Schneidelektrode im Koagulationsmodus mit einer Spannung zu beaufschlagen, die niedriger ist als die Koagulationsspannung, um dadurch jede Schneidwirkung auszuschließen. Damit kann der Leistungsschalter in betätigtem Zustand die Schneidelektrode mit einer Minderspannungsquelle verbinden, die eine verminderte Spannung bereitstellt. Diese mindere Spannung kann z.B. einen Bruchteil der Koagulationsspannung betragen, bspw. 75% oder 50% derselben. Die Minderspannungsquelle kann ein primärseitig von der Koagulationsspannung gespeister Transformator sein. Der Transformator kann als Spartransformator ausgebildet sein, so dass die Minderspannungsquelle von einer Anzapfung einer Wicklung gebildet wird, die mit der Koagulationsspannung beaufschlagt ist.

Es ist auch möglich, dass der Leistungsschalter die Schneidelektrode im aktivierten Zustand des Koagulationsaktivierungsschalters über ein Strombegrenzungsmittel mit der Schneidspannungsquelle oder mit der Koagulationsspannungsquelle verbindet. Das Strombegrenzungsmittel kann ein Kondensator, ein ohmscher Widerstand, ein nichtlinearer Widerstand, ein induktives Bauelement oder eine Kombination aus zwei oder mehreren der genannten Bauelemente sein. Jedenfalls ist es so bemessen, dass es den zur Schneidelektrode geleiteten Strom in dem Koagulationsmodus auf einen Wert begrenzt, der nicht zum Schneiden von Gewebe führt.

Alternativ ist es auch möglich, die Schneidelektrode im Koagulationsmodus potentialfrei zu schalten, d.h. mit keiner elektrischen Quelle zu verbinden. In diesem Fall nimmt die Schneidelektrode am Koagulationsprozess nicht teil, so dass im Bereich der Schneidelektrode eine geringe oder keine Gewebeschrumpfung eintritt.

Generell ist es vorteilhaft, wenn die Schneidelektrode und ein eventuell vorhandenes Widerlager in Bezug aufeinander federnd nachgiebig ausgebildet sind. Beispielsweise kann die Schneidelektrode federnd gelagert sein, um in Schlussrichtung der Branche beweglich zu sein. Zusätzlich oder alternativ kann das Widerlager beweglich angeordnet oder elastisch ausgebildet sein, um gleichfalls in Bewegungsrichtung der Branche bei schließendem Werkzeug der Schneidelektrode ausweichen zu können. Dies kann bei allen vorstehend erläuterten Ausführungsformen der elektrischen Schaltung Anwendung finden. Besonders vorteilhaft ist dies jedoch bei potentialfrei geschalteter Schneidelektrode. Hier ist es möglich im Koagulationsmodus ohne jegliche Schneidwirkung einen wenig geschrumpften Gewebestreifen zwischen zwei Versiegelungssäumen zu erzeugen, die ihrerseits von den Koagulationselektroden erzeugt wurden.

Das Koagulationsinstrument kann einen Schneidaktivierungsschalter enthalten, der dazu dient, die Schneidspannungsquelle direkt oder indirekt zu aktivieren. Zur indirekten Aktivierung ist er beispielsweise steuernd mit einer HF-Ausgangsspannungsquelle eines Generators verbunden, der eine geeignete HF-Ausgangsspannung zum Betrieb des Instruments abgibt. Diese HF-Ausgangsspannung kann eine Schneidspannung oder eine Koagulationsspannung sein. Ein in dem Instrument vorhandener Transformator kann als Schneidspannungsquelle dienen und von der Koagulationsspannung gespeist werden. Alternativ kann der Transformator auch von der Schneidspannung gespeist werden und die Koagulationsspannung liefern. Wird der Steuerschalter zur Aktivierung des Schneidmodus betätigt, ist der im Ruhezustand befindliche Leistungsschalter geschlossen und verbindet somit die Schneidspannungsquelle mit der Schneidelektrode.

Vorzugsweise sind der Schneidaktivierungsschalter und der Koagulationsaktivierungsschalter gegeneinander verriegelt, so dass sie lediglich alternativ betätigt werden können. Beispielsweise können die beiden Schalter ein gemeinsames Betätigungselement zum Beispiel in Gestalt einer Schaltwippe aufweisen. Diese stellt sicher, dass nur entweder der Schneidaktivierungsschalter oder der Koagulationsaktivierungsschalter betätigt wird.

Weitere Einzelheiten vorteilhafter Ausführungsformen der Erfindung sind Gegenstand der Zeichnung, der Beschreibung oder von Ansprüchen. Es zeigen:
Figur 1 eine Koagulations- und Dissektionseinrichtung bestehend aus einem Koagulations- und Dissektionsinstrument und einem speisenden Gerät, an das das Instrument mittels eines Kabels angeschlossen ist, in schematischer Darstellung,
Figur 2 das Werkzeug des Instruments nach Figur 1, in perspektivischer teilweiser geschnittener Darstellung,
Figur 3 und 4 das Instrument nach Figur 2 mit und ohne biologisches Gewebe, in Querschnittsdarstellung,
Figur 5 bis 10 verschiedene Ausführungsformen der elektrischen Schaltung zur Speisung der Elektroden des Instruments.

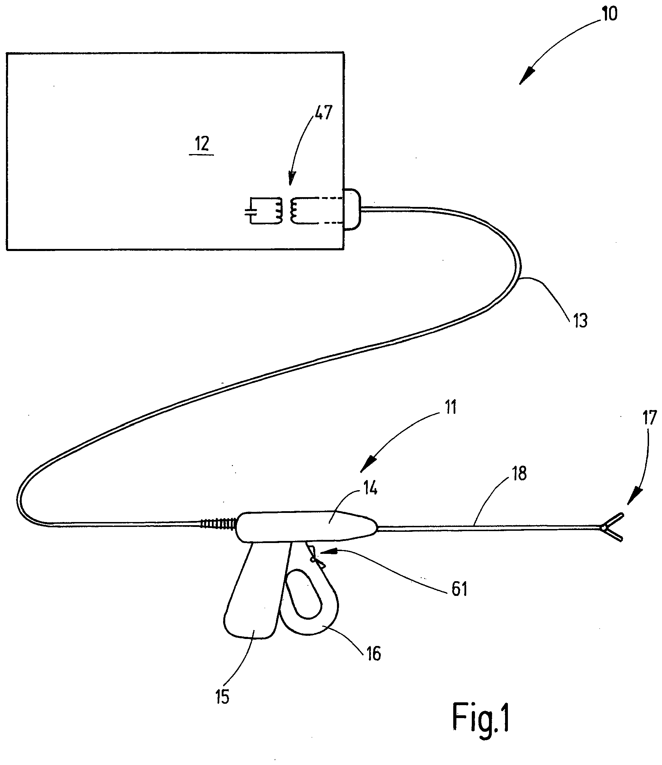

In Figur 1 ist eine Koagulationseinrichtung 10 veranschaulicht, zu der ein Koagulations- und Dissektionsinstrument 11 zur laparoskopischen Chirurgie und ein zur Speisung desselben dienendes Gerät 12 gehören. Das Instrument 11 ist mit dem Gerät 12 über ein mehradriges Kabel 13 verbunden, das elektrischen Strom zur Koagulation und/oder Dissektion von biologischem Gewebe von dem Gerät 12 zu dem Instrument 11 leitet und das außerdem wenigstens eine Signalleitung enthalten kann, um Befehle zum Freigeben und Sperren der elektrischen Energie von dem Instrument 11 zu dem Gerät 12 zu leiten.

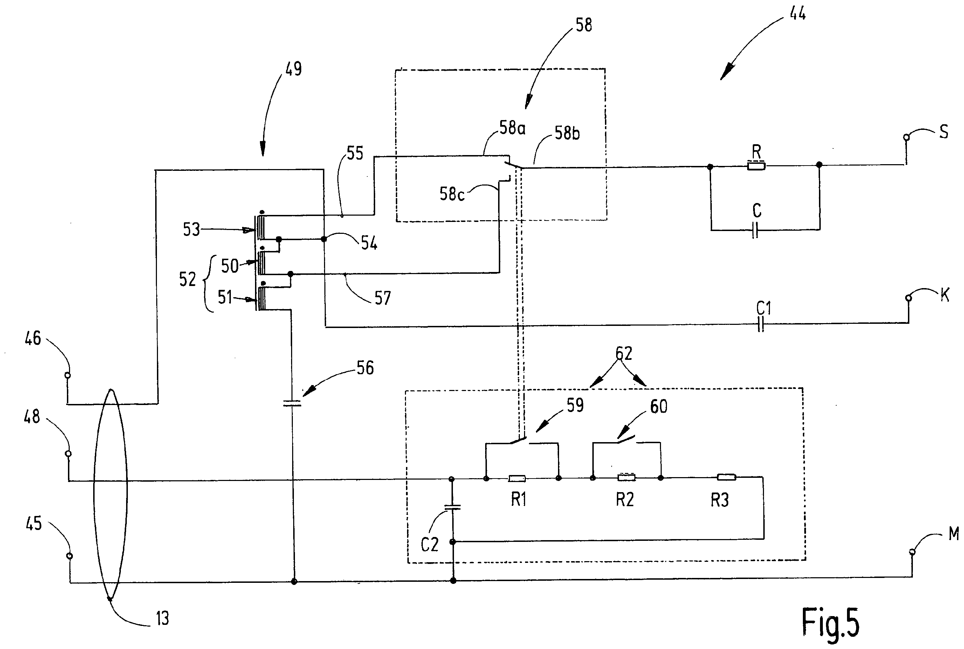

Das Instrument 11 weist ein Gehäuse 14 mit Handgriff 15 und Bedienelement 16 zur Betätigung eines Werkzeugs 17 auf, das an dem distalen Ende eines sich von dem Gehäuse 14 weg erstreckenden Schafts 18 angeordnet ist. Die Erfindung kann auch an einem Instrument zur offenchirurgischen Anwendung verwirklicht sein. Ein Schaft 18 eines solchen Instruments kann dann im Aufbau und Durchmesser vom Instrument gemäß Figur 1 abweichen und eine elektrische Schaltung gemäß den Figuren 5 bis 10 aufweisen. Außerdem kann die Erfindung an einem Instrument 11 realisiert sein, dessen Werkzeug 17 durch andere Mittel, beispielweise durch Steuermittel eines Roboters oder Schenkel einer Schere oder Zange, die direkt an der Gelenkachse des Werkzeugs 17 wirken, gesteuert wird. Bei solch einem Instrument 11 können dann das Gehäuse 14, der Handgriff 15 und das Bedienelement 16 zum Teil wegfallen bzw. anders gestaltet sein.

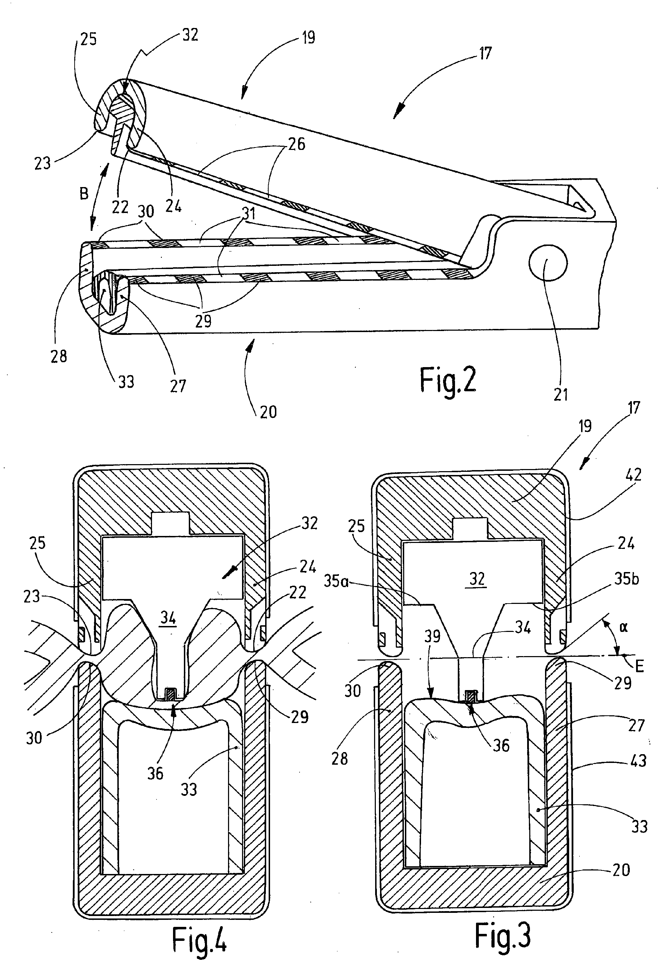

Das Werkzeug 17 ist in Figur 2 etwas detaillierter veranschaulicht. Es ist nach Art einer Zange ausgebildet und weist dazu eine erste Branche 19 und eine zweite Branche 20 auf, die an einem Scharnier 21 oder sonstigem Gelenk so mit einander verbunden sind, dass wenigstens eine der Branchen 19, 20 auf die andere Branche (20 oder 19) hin und von dieser weg beweglich ist. Die Öffnungs- und Schließbewegung der Branchen 19, 20 wird durch das Bedienelement 16 bzw. zwei Handbranchen bei z.B. einem offenchirurgischen Instrument gesteuert.

Die erste Branche 19 weist mindestens eine erste Koagulationselektrode auf. Im Ausführungsbeispiel sind zwei erste Koagulationselektroden 22, 23 vorgesehen, die an den äußersten Enden zweier Schenkel 24, 25 der im Querschnitt u-förmig ausgebildeten ersten Branche 19 vorgesehen sind. Die ersten Koagulationselektroden 22, 23 sind vorzugsweise elektrisch untereinander verbunden. Außerdem können sie längs der freien Enden jedes Schenkels 24, 25 durch isolierende Abschnitte 26 unterbrochen sein.

Die zweite Branche 20 ist entsprechend ausgebildet. Sie weist ebenfalls vorzugsweise einen U-förmigen Querschnitt mit zwei Schenkeln 27, 28 auf, an deren freien Enden zweite Koagulationselektroden 29, 30 ausgebildet sind. Die Koagulationselektroden 29, 30 beziehungsweise deren Koagulationsflächen sind in einem Winkel a zu einer gedachten Ebene E (Figur 3) angeordnet, die in Richtung der Längsachse der Branche 20 verläuft und auf beiden Schenkeln 27 und 28 aufliegt. Der Winkel a zu der Ebene E beträgt vorzugsweise 20 Grad. Die Koagulationsflächen der Koagulationselektroden 29, 30 sind vorzugsweise zu der Auflagefläche 39 hin abfallend angeordnet. Die Koagulationselektroden 29, 30 können wiederum durch isolierende Abschnitte 31 unterbrochen sein, um Kurzschluss zwischen den beiden Branchen 19, 20 zu verhindern, wenn diese aufeinander zu bewegt und miteinander in Anlage gebracht werden.

Während eine Branche, beispielsweise die erste Branche 19, in einer mittleren Nut einen Schneidelektrodeneinsatz 32 aufnimmt, ist gegenüber liegend in einer entsprechenden Nut der zweiten Branche 20 ein Widerlagerelement 33 angeordnet (Figur 3). Der Schneidelektrodeneinsatz 32 ist aus einem nichtleitenden Material, vorzugsweise Kunststoff, Silikon oder Keramik ausgebildet. Er weist einen mittleren, sich zu seinem Fuß hin verdickenden Wandabschnitt 34 auf, der von einer Begrenzungsfläche 35a, 35b aufragt, die sich jeweils von dem Schenkel 25 zu dem Wandabschnitt 34 und von dem Wandabschnitt 34 zu den Schenkel 24 erstreckt. Die Begrenzungsflächen 35a, 35b liegen in einer gemeinsamen Ebene und stehen rechtwinklig zu dem Wandabschnitt 34 wie auch rechtwinklig zu den Schenkel 24, 25. Die Schenkel 24, 25 überragen die Begrenzungsflächen 35a, 35b.

An der von den Begrenzungsflächen 35a, 35b weg weisenden und dazu parallelen Fläche ist der Wandabschnitt 34 mit einer Schneidelektrode 36 versehen, die zur bedarfsweisen Durchtrennung von biologischem Gewebe dient. Die aus einem elektrisch gut leitfähigen Material bestehende Schneidelektrode 36 ist in den aus isolierendem Material bestehenden Wandabschnitt 34 eingebettet und liegt nur an der dem Widerlagerelement 33 zugewandten Seite frei. In geschlossenem Zustand des Werkzeugs 17 findet die Schneidelektrode 36 Anlage an dem Widerlagerelement 33, das eine sich von einem Schenkel 28 zu dem anderen Schenkel 27 erstreckende Gewebeauflagefläche 39 aufweist. Das Widerlagerelement 33 besteht z.B. aus einem Elastomer oder einem sonstigen elektrisch isolierenden vorzugsweise federnd nachgiebigem Material, z.B. Silikon.

Die Branchen 19, 20 können, wie Figur 3 andeutet, an ihren Außenseiten jeweils mit einer Isolierung 42, 43 versehen sein, um elektrischen Kontakt zu umliegendem Gewebe zu unterbinden.

Zur elektrischen Versorgung des Werkzeugs 17 dient in Verbindung mit dem Gerät 12 eine elektrische Schaltung 44, die in dem Instrument 11, insbesondere in dem Gehäuse 14 desselben untergebracht sein kann. Die elektrische Schaltung 44 ist in Figur 5 beispielhaft veranschaulicht. Die Schaltung 44 weist drei Ausgangsanschlüsse M, K und S auf, die mit den Elektroden verbunden sind. Im Einzelnen dient der Anschluss M als Masseanschluss und ist vorzugsweise mit der zweiten Branche 20 und entsprechend mit den zweiten Koagulationselektroden 29, 30 verbunden. Damit bilden die Koagulationselektroden 29, 30 den gemeinsamen elektrischen Gegenpol für die Schneidelektrode 36 und die ersten Koagulationselektroden 22, 23. Der Ausgangsanschluss K ist ein Koagulationsanschluss der vorzugsweise mit der ersten Branche 19 und somit mit den ersten Koagulationselektroden 22, 23 verbunden ist. Der Ausgangsanschluss S ist der Anschluss für Schneidspannung. Dieser Anschluss ist mit der Schneidelektrode 36 verbunden.

Eingangsseitig führen Leitungen durch das Kabel 13 zu dem mit dem Gerät 12 verbundenen Stecker, der mindestens drei Anschlüsse aufweist. Zwei Anschlüsse 45, 46 dienen zum Anschluss an eine HF-Ausgangsspannungsquelle 47 (schematisch angedeutet in Figur 1) die als HF-Generator ausgebildet ist und elektrische Energie zum Betrieb des Instruments 11 bereitstellt. Ein dritter Eingangsanschluss 48 dient zum Aktivieren oder Deaktivieren der HF-Ausgangsspannungsquelle 47. Das Instrument enthält einen Transformator 49, der als Spartransformator ausgebildet sein kann. Er kann eine auf zwei Teilwicklungen 50, 51 aufgeteilte Primärwicklung 52 aufweisen, sowie eine Sekundärwicklung 53, die nach dem Spartrafoprinzip mit der Primärwicklung 52 in Reihe geschaltet ist. Andere Transformatoranordnungen mit getrennten Primär- und Sekundärwicklungen sind jedoch ebenfalls möglich.

Der Eingangsanschluss 46 ist mit der Primärwicklung 52 verbunden, wobei dieser Anschluss der Primärwicklung 52 zugleich eine Koagulationsspannungsquelle 54 darstellt. Das obere Ende der Sekundärwicklung 53 stellt hingegen eine Schneidspannungsquelle 55 dar. Das untere Ende der Primärwicklung 52 ist direkt oder, falls gewünscht, über einen Koppelkondensator 56 mit Masse, d.h. dem Eingangsanschluss 45 bzw. dem Ausgangsanschluss M verbunden. Der zwischen den Teilwicklungen 50, 51 herausgeführte Anschluss des Transformators 49 kann als Minderspannungsquelle 57 dienen. Vorzugsweise verhalten sich die Windungszahlen der ersten Teilwicklung 50 zur zweiten Teilwicklung 51 zur Sekundärwicklung 53 wie 11:11:77. Damit wird eine an dem Eingangsanschluss 46 anliegende Versorgungsspannung von zum Beispiel 100 V (HF) (gemessen gegen Masse, d.h. Eingangsanschluss 45) als Koagulationsspannung an den die Koagulationsspannungsquelle bildenden Punkt weitergegeben während die Schneidspannungsquelle 55 etwa 450V gemessen gegen Masse liefert.

Von der Schneidspannungsquelle 55 verläuft ein Leitungspfad zum Ausgangsanschluss S, d.h. zu der Schneidelektrode 36. In diesem Strompfad können ein Kondensator (vorzugsweise mehrere Nanofarad) und ein Widerstand (mehrere 10 k2) vorgesehen sein, um eine Funkenbildungserkennung zu ermöglichen. Eine Funkenbildung würde zu einem Gleichanteil des Stroms an dem Anschluss 46 führen, was geräteseitig erfassbar ist. Ist eine Funkenerkennung nicht gewünscht, können der Widerstand R, sowie der Kondensator 56 auch entfallen. Des Weiteren liegt in dem Strompfad zwischen der Schneidspannungsquelle 55 und der Schneidelektrode ein Leistungsschalter 58 mit einem beweglichen Kontakt 58b und mindestens einem festen Kontakt 58a, 58c. Der Leistungsschalter 58 verbindet in der vorliegenden ersten Ausführungsform in nichtbetätigtem Zustand die Schneidspannungsquelle 55 mit der Schneidelektrode. In betätigtem Zustand verbindet der Leistungsschalter 58 hingegen die Schneidelektrode 36 mit einer anderen Spannung, beispielsweise mit der Minderspannungsquelle 57.

Der Ausgangsanschluss K für die Koagulationsspannung ist mit einer der Branchen, vorzugsweise der ersten Branche 19 verbunden, die auch den Schneidelektrodeneinsatz 32 beherbergt. In dem entsprechenden Strompfad kann ein Kondensator Cl vorgesehen sein. Dieser kann zur Begrenzung des Koagulationsstroms dienen und hat vorzugsweise einen Wert von mehreren nF. Der Koagulationsstrom hat vorzugsweise eine Frequenz zwischen 300 kHz und 400 kHz und liegt als CW (Dauerstrich, d.h. ununterbrochene HF-Spannung) an.

Die Schaltung 44 enthält vorzugsweise einen Aktivierungsschalter 62, der ein Koagulationsaktivierungsschalter 59 oder ein Schneidaktivierungsschalter 60 sein kann. Beide Schalter 59, 60 können von einem gemeinsamen Betätigungselement 61 aus betätigt werden, beispielsweise durch eine Wippe (Figur 1), so dass die Betätigung des Aktivierungsschalters 62 entweder den Schneidaktivierungsschalter 59 oder den Koagulationsaktivierungsschalter 60 jedoch nicht beide gleichzeitig schließt. In der Mittelstellung der Wippe ist keiner der beiden Schalter 59, 60 geschlossen. Die Schalter 59, 60 können parallel zu Widerständen R1, R2 angeordnet sein, die mit einem dritten Widerstand R3 in Reihe geschaltet sind. Die Reihenschaltung der Widerstände R1 bis R3 ist vorzugsweise zwischen den Eingangsanschlüssen 48, 45 vorgesehen. Zur Vermeidung von Störungen können die Widerstände R1, R2, R3 einzeln oder in Reihenschaltung durch einen oder mehrere Kondensatoren C2 überbrückt sein.

Eine Überbrückung eines der beiden Widerstände R1 oder R2 wird von dem an den Anschluss 48 angeschlossenen Gerät erfasst und als Befehl zur Aktivierung der HF-Ausgangsspannungsquelle 47 interpretiert. Diese wird somit sowohl bei der Betätigung des Koagulationsaktivierungsschalters 59 wie auch bei der Betätigung des Schneidaktivierungsschalters 60 aktiviert. Sind beide Schalter 59, 60 offen ist die HF-Ausgangsspannungsquelle 47 deaktiviert.

Eine Besonderheit liegt in der mechanischen Verbindung zwischen dem Koagulationsaktivierungsschalter 59 und dem Leistungsschalter 58. Diese sind so verbunden, dass bei einer entsprechenden Betätigung des Betätigungselements 61 zunächst der Leistungsschalter 58 die Verbindung zwischen der Schneidspannungsquelle 55 und dem Ausgangsanschluss S bzw. der Schneidelektrode 36, d.h. zwischen den Kontakten 58a und 58b, auftrennt, bevor der Koagulationsaktivierungsschalter 59 schließt. Der Leistungsschalter 58 arbeitet dabei vorzugsweise hysteresefrei. Der Koagulationsaktivierungsschalter 59 kann hingegen eine deutlich spürbare Schalthysterese aufweisen, um den Bediener eine taktile Rückmeldung über die Aktivierung des Koagulationsmodus oder des Schneidmodus zu geben. Der Hysteresebereich der Bewegung des Schalters ist dabei so bemessen, dass die Umschaltung des Leistungsschalters 58 außerhalb des Hysterebereichs des Koagulationsaktivierungsschalters 60 erfolgt.

Die insoweit beschriebene Einrichtung 10 arbeitet wie folgt:
Im Betrieb ist das Instrument 11 an das Gerät 12 angeschlossen. Der Bediener kann dann mit dem Instrument 11 Gewebe erfassen und durch Betätigung des Bedienelements 16 die Branchen 19, 20 schließen. Er kann beispielsweise ein Hohlorgan, z.B. ein Blutgefäß fassen, das dann, wie Figur 4 zeigt, zwischen den ersten Koagulationselektroden 22, 23 und den zweiten Koagulationselektroden 29, 30 gefasst und eingeklemmt ist. Soll nun eine Koagulation und eine Dissektion gleichzeitig durchgeführt werden, wird der Schneidaktivierungsschalter 60 betätigt. Das Instrument 11 erhält nun eine Versorgungsspannung, die als Koagulationsspannung an der Koagulationsspannungsquelle 54 für die Koagulationselektroden 22, 23 bereitgestellt wird. Zugleich erzeugt der Transformator 49 die Schneidspannung, die an dem Transformatorausgang als Schneidspannungsquelle 55 bereitgestellt und über den Leistungsschalter 58 an die Schneidelektrode 36 geleitet wird.

Entschließt sich der Bediener von einer Dissektion abzusehen und lediglich eine Koagulation durchzuführen, betätigt er hingegen den Koagulationsaktivierungsschalter 59. Dabei schaltet er zunächst den Leistungsschalter 58 um, so dass die Schneidelektrode 36 mit der Minderspannungsquelle 57 verbunden wird. Erst danach schließt der Koagulationsaktivierungsschalter 59 und aktiviert dadurch die Versorgungsspanungsquelle 47. Somit gelangt Koagulationsspannung aus der Koagulationsspannungsquelle 54 an den Anschluss K, d.h. die Koagulationselektroden 22, 23, während die Schneidelektrode 36 eine mindere Spannung von zum Beispiel lediglich der halben Koagulationsspannung erhält. Damit werden Schneideffekte an der Schneidelektrode 36 vermindert oder vermieden.

Bei der vorgestellten Schaltung 44 sind zahlreiche Abwandlungen möglich, die nachfolgend unter Beschränkung auf die Unterschiede zur Schaltung 44 gemäß Figur 5 beschrieben werden:

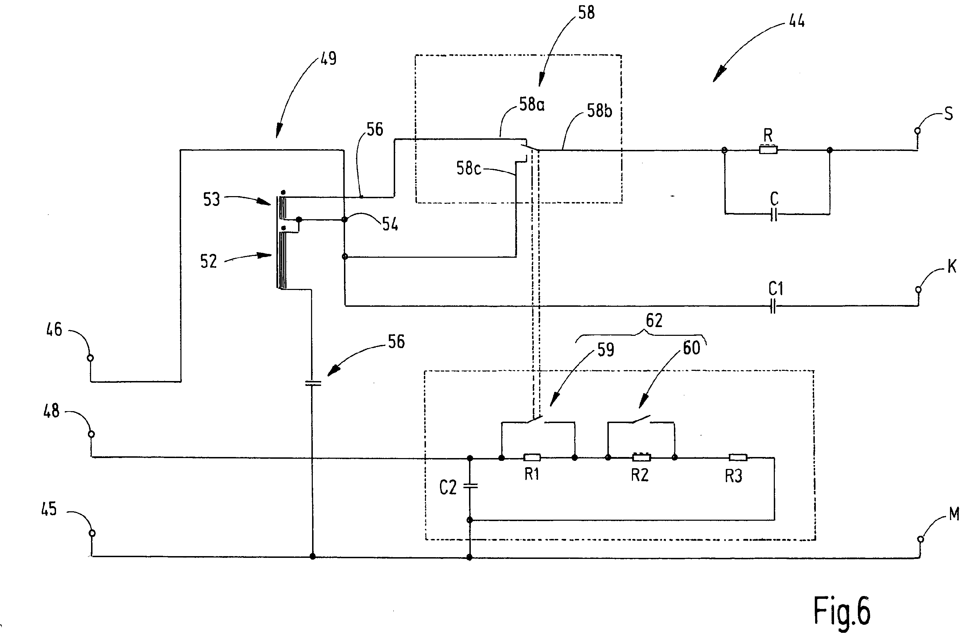

Die Schaltung 44 nach Figur 6 weist einen Transformator 49 mit nicht aufgeteilter Primärwicklung 52 auf. Somit enthält die Schaltung 44 keine Minderspannungsquelle. Während der Kontakt 58a des Leistungsschalters 58 wie vorstehend mit dem ersten Ende der Sekundärwicklung 53, d.h. mit der Schneidspannungsquelle 55 verbunden ist, ist der Kontakt 58c des Leistungsschalters 58 mit der Koagulationsspannungsquelle 54 verbunden. Bei dieser Ausführungsform führt eine Betätigung des Koagulationsaktivierungsschalters 59 wie vorstehend beschrieben, zu einer Trennung des Schneidstrompfads vor Aktivierung der HF-Ausgangsspannungsquelle 47. Ein Freigeben des Koagulationsaktivierungsschalters 59 führt zu einer Deaktivierung der HF-Ausgangsspannungsquelle 47 bevor der Schneidstrompfad von dem Schalter 58 wieder geschlossen wird. Ansonsten gilt die zu den Figuren 1 bis 5 gegebene Beschreibung unter Zugrundelegung der bereits eingeführten Bezugszeichen entsprechend.

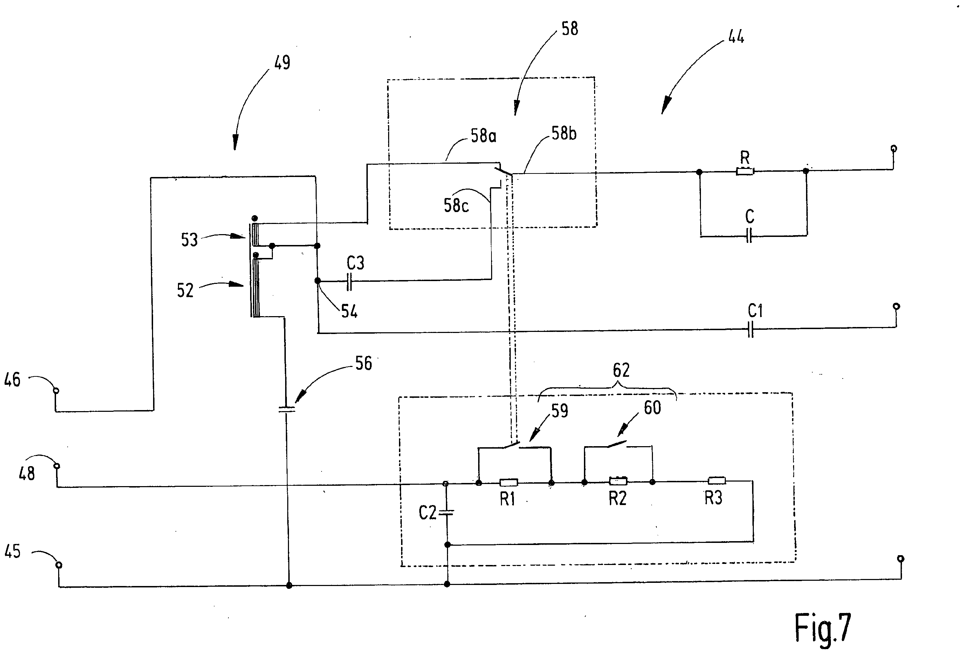

Gemäß Figur 7 ist es aufbauend auf der Ausführungsform nach Figur 6 möglich, bei der Schaltung 44 in dem von der Koagulationsspannungsquelle 54 zu dem Kontakt 58c des Leistungsschalters 58 führenden Pfad ein strombegrenzendes Bauelement anzuordnen. Dieses kann beispielsweise ein Kondensator C3 oder auch ein anderes Bauelement, beispielsweise eine Spule (Induktivität), ein Widerstand oder eine Kombination aus zwei oder mehreren solchen Bauelementen sein. Die Impedanz des Kondensators C3 oder eines sonstigen strombegrenzenden Bauelements ist dabei so bemessen, dass der im Koagulationsmodus an die Schneidelektrode 36 gelangende Strom begrenzt wird und somit keine Schneidwirkung mehr hat. Ansonsten gilt die zu den Figuren 1 bis 5 gegebene Beschreibung unter Zugrundelegung der bereits eingeführten Bezugszeichen entsprechend.

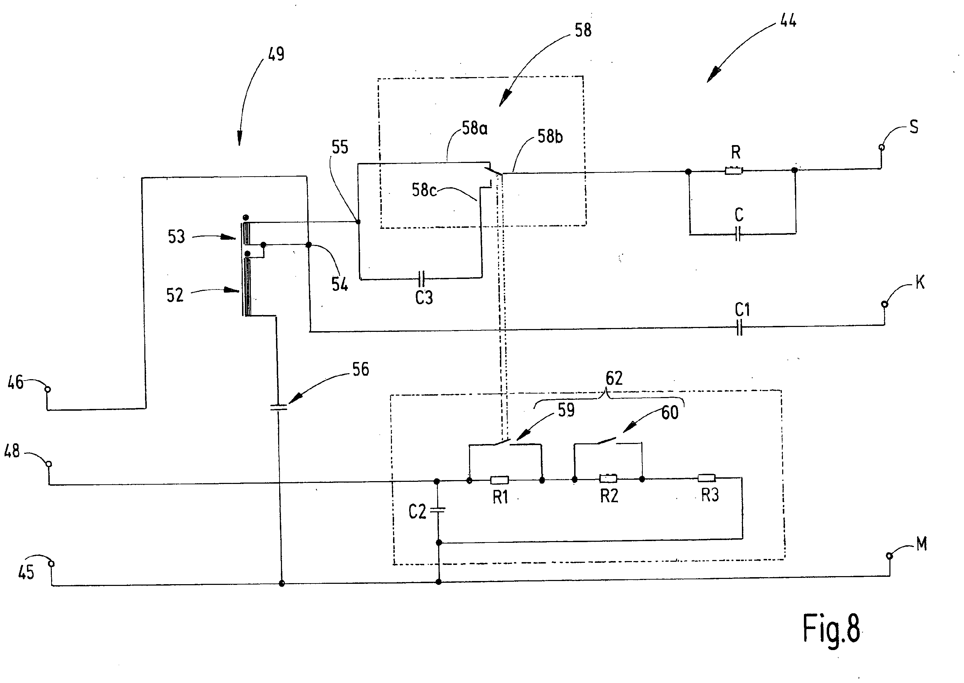

Alternativ kann, wie die Schaltung 44 nach Figur 8 zeigt, der Kontakt 58c des Leistungsschalters 58 über das strombegrenzende Bauelement, beispielsweise den Kondensator C3, auch mit der Schneidspannungsquelle 55 verbunden sein. Im Koagulationsmodus liegt somit an der Schneidelektrode 36 zunächst Schneidspannung an, wobei aber der Strom auf Werte begrenzt wird, die keine Schneidwirkung oder keine nennenswerte Schneidwirkung mehr gestatten. Ansonsten gilt die zu den Figuren 1 bis 5 gegebene Beschreibung unter Zugrundelegung der bereits eingeführten Bezugszeichen entsprechend.

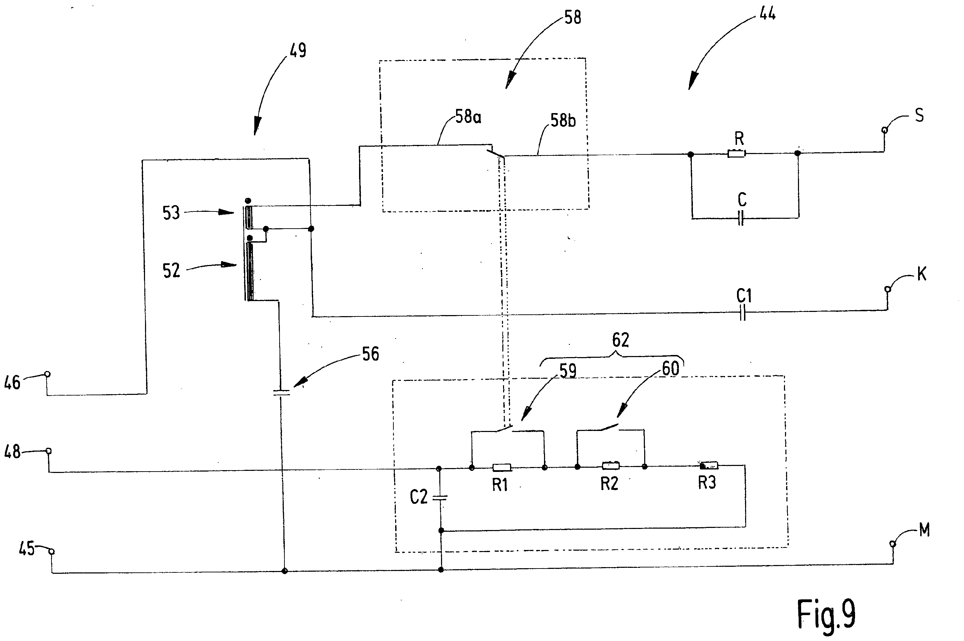

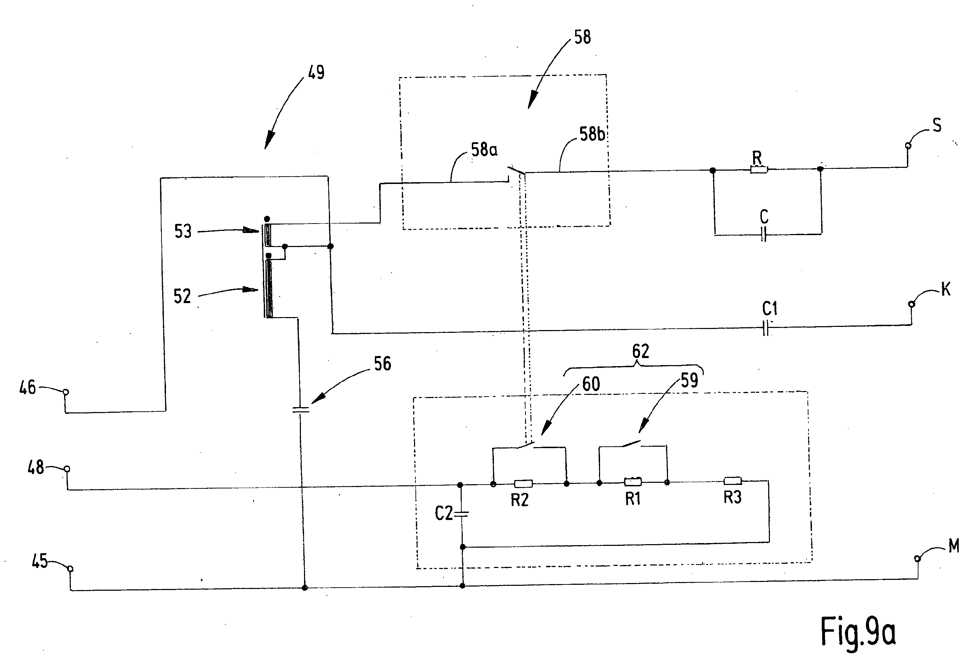

Wie Figur 9 zeigt, ist es darüber hinaus möglich, den Leistungsschalter 58 nicht als Umschalter, wie es bei allen vorstehenden Ausführungsformen der Fall ist, sondern als Öffner auszubilden (d.h. Kontakt 58c fehlt). Der Öffner ist wiederum voreilend zu dem Koagulationsaktivierungsschalter 59 ausgebildet, d.h. er öffnet bevor der Koagulationsaktivierungsschalter 59 schließt und er schließt erst nachdem der Koagulationsaktivierungsschalter 59 geöffnet hat. In diesem Fall ist die Schneidelektrode 36 im Koagulationsmodus potentialfrei und somit stromlos. Ansonsten gilt die zu den Figuren 1 bis 5 gegebene Beschreibung unter Zugrundelegung der bereits eingeführten Bezugszeichen entsprechend.

Eine weitere mögliche bevorzugte Abwandlung der erfindungsgemäßen Schaltung ist aus Figur 9a ersichtlich. Bei dieser Ausführungsform ist der Leistungsschalter 58 ein Schließer, der bei Aktivierung des Schneidaktivierungsschalters 60 eine leitende Verbindung zwischen den Kontakten 58a, 58b herstellt. Der Leistungsschalter ist beim Schließen dem Schneidaktivierungsschalter 60 voreilend und beim Deaktivieren nacheilend zum Schneidaktivierungsschalter 60. Wird hingegen nur der Koagulationsaktivierungsschalter 59 betätigt, bleibt der Leistungsschalter 58 offen. Die Schneidelektrode kann stromlos sein oder über die parasitäre Kapazität des Leistungsschalters 58 einen vernachlässigbar geringen Strom führen. Ansonsten gilt die zu den Figuren 1 bis 5 gegebene Beschreibung unter Zugrundelegung der bereits eingeführten Bezugszeichen entsprechend.

Bei allen Ausführungsformen der Figuren 5, 6, 7, 8 und 10 ist es auch möglich, dass die mechanische Verbindung anstelle zwischen dem Leistungsschalter 58 und dem Koagulationsaktivierungsschalter 59 alternativ zwischen dem Leistungsschalter 58 und dem Schneidaktivierungsschalter 60 besteht. Im Ruhezustand ist dann der Kontakt 58b mit dem Kontakt 58c verbunden. Wird nun der Schneidaktivierungsschalter 60 betätigt, schaltet der Leistungsschalter 58 voreilend um, um die Verbindung zwischen dem Kontakt 58a und dem Kontakt 58b vor Schließen des Schneidaktivierungsschalters 60 herzustellen. Bei Deaktivierung schaltet der Leistungsschalter 58 nacheilend um. Bei Betätigung des Koagulationsaktivierungsschalters 59 bleibt der Leistungsschalter 58 im Ruhezustand und damit der Kontakt 58b weiter mit dem Kontakt 58c verbunden.

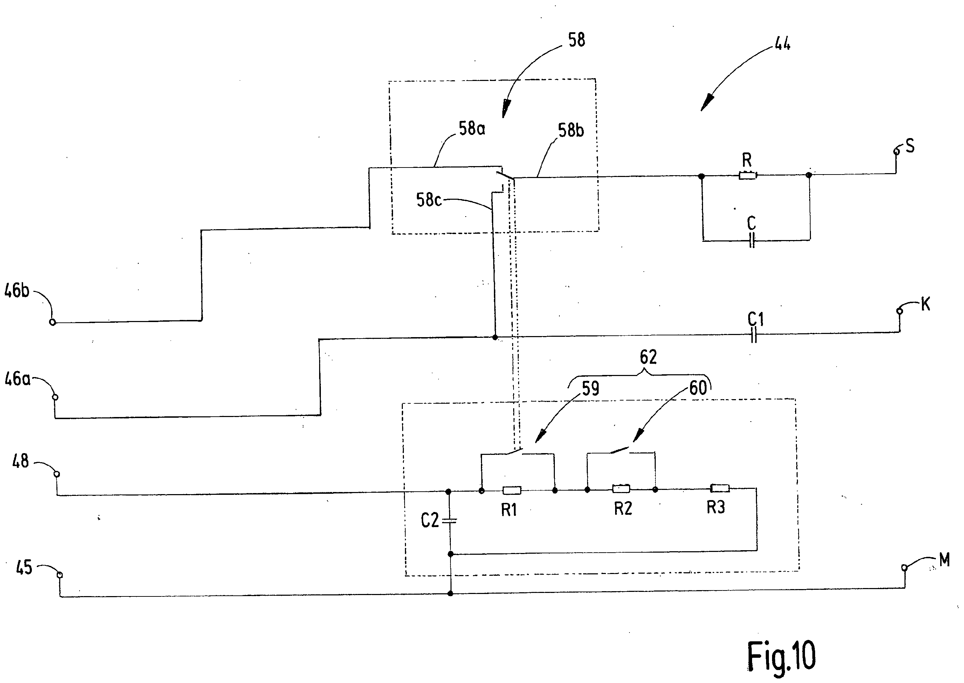

Während bei allen vorstehend beschriebenen Ausführungsformen der Schaltung 44 die Koagulationsspannungsquelle 54 und die Schneidspannungsquelle 55 Teil der Schaltung 44 waren, ist es auch möglich, die beiden Spannungsquellen in dem Gerät 12 vorzusehen. Anstelle eines zur Verbindung mit einer HF-Ausgangsspannungsquelle 47 dienenden Anschluss 46 sind an dem Stecker zwei Anschlüsse 46a, 46b vorgesehen, siehe Figur 10. Hierbei ist der Anschluss 46a an eine in dem Gerät 12 vorgesehene Koagulationsspannungsquelle und der Anschluss 46b an eine in dem Gerät 12 vorgesehene Schneidspannungsquelle angeschlossen. Beide werden durch Betätigung eines der Schalter 59, 60 gleichzeitig aktiviert, wobei der Leistungsschalter 58 wie vorstehend beschrieben in der gleichen Schaltreihenfolge zu dem Koagulationsaktivierungsschalter 59 geschaltet wird und somit in Koagulationsmodus jede Schneidwirkung unterdrückt. Alternativ kann der Leistungsschalter 58 auch mit dem Schneidaktivierungsschalter 60 verbunden bzw. gekoppelt sein und im Leistungsschalter 58 im Ruhezustand der Kontakt 58b mit dem Kontakt 58c verbunden sein (nicht dargestellt). In diesem alternativen Aufbau wird bei Betätigung des Schneidaktivierungsschalters 60 der Leistungsschalter 58 voreilend umgeschaltet sodass im Schneidemodus die Schneidwirkung zugelassen wird. Beim Lösen den Schneidaktivierungsschalters 60 stellt sich nacheilend der Leistungsschalter 58 wieder zurück in den Ruhezustand, sodass bei einer folgenden Koagulationsaktivierung die Schneidwirkung unterdrückt bleibt. Ansonsten gilt die zu den Figuren 1 bis 5 gegebene Beschreibung unter Zugrundelegung der bereits eingeführten Bezugszeichen entsprechend.

Die erfindungsgemäße Koagulationseinrichtung 10 enthält ein Instrument 11, dessen Werkzeug 17 Koagulationselektroden 22, 23, 27, 28 sowie eine Schneidelektrode 36 aufweist. Die Elektroden werden von einer Schaltung 44 gespeist, die eine Koagulationsspannungsquelle 54 und eine Schneidspannungsquelle 55 enthält oder an solche Quellen anschließbar ist. Das Gerät weist einen Schneidaktivierungsschalter 60 und einen Koagulationsaktivierungsschalter 59 auf. Erster ist mit einem Leistungsschalter 58 verbunden, der im Koagulationsmodus den Strompfad von der Schneidspannungsquelle 55 zu der Schneidelektrode 36 trennt, bevor die Koagulationsspannungsquelle 54 aktiviert wird. Damit ist dem Anwender auf einfache Weise ein verlässliches Mittel an die Hand gegeben, um komplizierte Operationen flexibel durchführen zu können.

Die erfindungsgemäße Koagulationseinrichtung (10) enthält ein Instrument (11), dessen Werkzeug (17) Koagulationselektroden (22, 23, 27, 28) sowie eine Schneidelektrode (36) aufweist. Die Elektroden werden von einer Schaltung (44) gespeist, die eine Koagulationsspannungsquelle (54) und eine Schneidspannungsquelle (55) enthält oder an solche Quellen anschließbar ist. Das Gerät weist einen Schneidaktivierungsschalter (60) und einen Koagulationsaktivierungsschalter (59) auf. Erster ist mit einem Leistungsschalter 58 verbunden, der im Schneidemodus den Strompfad von der Schneidspannungsquelle (55) zu der Schneidelektrode (36) verbindet, bevor die Schneidspannungsquelle 55 bzw. die HF-Ausgangsspannungsquelle 47 und die Koagulationsspannungsquelle 54 aktiviert wird. Damit ist dem Anwender auf einfache Weise ein verlässliches Mittel an die Hand gegeben, um komplizierte Operationen flexibel durchführen zu können.

**Bezugszeichen**

| | |
|---|---|
| 10 | Koagulationseinrichtung |
| 11 | Instrument zur Koagulation und Dissektion von biologischem Gewebe |
| 12 | Gerät |
| 13 | Kabel |
| 14 | Gehäuse |
| 15 | Handgriff |
| 16 | Bedienelement |
| 17 | Werkzeug |
| 18 | Schaft |
| 19 | erste Branche des Werkzeugs 17 |
| 20 | zweite Branche des Werkzeugs 17 |
| 21 | Scharnier |
| 22, 23 | erste Koagulationselektroden |
| 24, 25 | Schenkel der ersten Branche 19 |
| 26 | isolierende Abschnitte |
| 27, 28 | Schenkel der zweiten Branche 20 |
| 29, 30 | zweite Koagulationselektroden |
| 31 | isolierende Abschnitte |
| 32 | Schneidelektrodeneinsatz |
| 33 | Widerlagerelement |
| 34 | mittlerer Wandabschnitt |
| 35a, 35b | Begrenzungsfläche |
| 36 | Schneidelektrode |
| | |
| | |
| 39 | Anlagefläche |
| | |
| 42, 43 | Isolierung |
| 44 | Schaltung |
| M, K, S | Ausgangsanschlüsse |
| 45, 46 | Eingangsanschlüsse der Schaltung 44 |
| 47 | HF-Ausgangsspannungsquelle (HF-Generator) |
| 48 | Eingangsanschluss |
| 49 | Transformator |
| 50, 51 | Teilwicklungen |
| 52 | Primärwicklung |
| 53 | Sekundärwicklung |
| 54 | Koagulationsspannungsquelle |
| 55 | Schneidspannungsquelle |
| 56 | Koppelkondensator |
| 57 | Minderspannungsquelle |
| C, C1, C2, C3 | Kondensatoren |
| R, R1, R2, R3 | Widerstände |
| 58 | Leistungsschalter |
| 58a | Fester Kontakt des Schalters 58 (Ruhekontakt) |
| 58b | beweglicher Kontakt des Schalters 58 |
| 58c | Fester Kontakt des Schalters 58 (Arbeitskontakt) |
| 59 | Koagulationsaktivierungsschalter |
| 60 | Schneidaktivierungsschalter |
| 61 | Betätigungselement |
| 62 | Aktivierungsschalter |

## Patentansprüche

1. Instrument zur Koagulation und bedarfsweisen Dissektion von biologischem Gewebe,
mit einem Werkzeug (17), das wenigstens eine erste Koagulationselektrode (22) und eine zweite Koagulationselektrode (29) aufweist, die relativ zueinander beweglich sind, um zwischen einander Gewebe zu fassen, und das wenigstens eine Schneidelektrode (36) aufweist, um zwischen der wenigstens einen ersten Koagulationselektrode (22) und der wenigstens einen zweiten Koagulationselektrode (29) gefasstes Gewebe zu durchtrennen,
mit einer elektrischen Schaltung (44), die an die Koagulationselektroden (22, 29) und an die Schneidelektrode (36) angeschlossen ist und eine Schneidspannungsquelle (55) sowie eine Koagulationsspannungsquelle (54) enthält oder an solche anschließbar ist, wobei die Schneidspannungsquelle (55) durch einen im Instrument (11) angeordneten Transformator (49) gebildet ist, der eingangsseitig an die HF-Ausgangsspannungsquelle (47) anschließbar oder angeschlossen ist, und wobei die HF-Ausgangsspannungsquelle (47) zur Speisung der Koagulationsspannungsquelle (54) dient,
wobei die Schaltung (44) drei Ausgangsanschlüsse (M, K und S) aufweist, von denen der eine Ausgangsanschluss (M) ein mit der wenigstens einen zweiten Koagulationselektrode (29) verbundener Masseanschluss ist, der eine weitere Ausgangsanschluss (K) ist ein mit der wenigstens einen ersten Koagulationselektrode (22) verbundener Koagulationsanschluss ist und der andere weitere Ausgangsanschluss (S) ein mit der wenigstens einen Schneidelektrode (36) verbundener Anschluss für eine Schneidspannung der Schneidspannungsquelle (55) ist,
wobei die elektrische Schaltung (44) einen Leistungsschalter (58) aufweist, der in einem Strompfad zwischen der Schneidspannungsquelle (55) und der Schneidelektrode (36) angeordnet und zur wahlweisen Trennung der Schneidelektrode (36) von der Schneidspannungsquelle (55) oder zur wahlweisen Verbindung der Schneidelektrode (36) mit der Schneidspannungsquelle (55) eingerichtet ist, und einen Aktivierungsschalter (62) aufweist, der zur wahlweisen Aktivierung der Koagulationsspannungsquelle (54), der Schneidspannungsquelle (55) oder der HF-Ausgangsspannungsquelle (47) eingerichtet ist, wobei der Aktivierungsschalter (62) und der Leistungsschalter (58) von einem gemeinsamen Betätigungselement (61) seriell gesteuert sind.

2. Instrument nach Anspruch 1, **dadurch gekennzeichnet, dass** die elektrische Schaltung (44) eingangsseitig drei Anschlüsse (45, 46, 48) aufweist, wobei zwei Anschlüsse (45, 46) zum Anschluss an die HF-Ausgangsspannungsquelle (47) eingerichtet sind, um elektrische Energie zum Betrieb des Instruments (11) bereitzustellen, und wobei ein dritter Anschluss (48) zum Aktivieren oder Deaktivieren der HF-Ausgangsspannungsquelle (47) eingerichtet ist.

3. Instrument nach Anspruch 1 oder 2, **dadurch gekennzeichnet, dass** der Leistungsschalter (58) und der Aktivierungsschalter (62) derart mit dem Betätigungselement (61) verbunden sind, dass bei einer Betätigung des Betätigungselements (61) der Leistungsschalter (58) schließt, bevor der Aktivierungsschalter (62) schließt, und dass bei einer Freigabe des Betätigungselements (61) der Aktivierungsschalter (62) öffnet bevor der Leistungsschalter (58) öffnet.

4. Instrument nach einem der vorstehenden Ansprüche, **dadurch gekennzeichnet, dass** der Leistungsschalter (58) im Ruhezustand keine elektrische Verbindung zwischen der Schneidspannungsquelle (55) und der Schneidelektrode (36) erbringt und in betätigtem Zustand die Schneidelektrode (36) mit der Schneidspannungsquelle (55) verbindet.

5. Instrument nach Anspruch 4, **dadurch gekennzeichnet, dass** der Leistungsschalter (58) im Ruhezustand die Schneidelektrode (36) mit der Koagulationsspannungsquelle (54) oder mit einer Minderspannungsquelle (57) verbindet oder potentialfrei geschaltet ist.

6. Instrument nach Anspruch 5, **dadurch gekennzeichnet, dass** die Minderspannungsquelle (57) ein primärseitig an die Koagulationsspannungsquelle (54) angeschlossener Transformator (49) ist.

7. Instrument nach Anspruch 4, **dadurch gekennzeichnet, dass** der Leistungsschalter (58) im Ruhezustand die Schneidelektrode (36) mit einem Strombegrenzungsmittel (C3) verbindet.

8. Instrument nach einem der vorstehenden Ansprüche, **dadurch gekennzeichnet, dass** das Instrument (11) einen Koagulationsaktivierungsschalter (59) aufweist, der zur Aktivierung der HF-Ausgangsspannungsquelle (47) eingerichtet ist.

9. Instrument nach einem der vorhergehenden Ansprüche, **dadurch gekennzeichnet, dass** das Instrument (11) einen Schneidaktivierungsschalter (60) aufweist, der zur Aktivierung der HF-Ausgangsspannungsquelle (47) eingerichtet ist.

10. Instrument nach Anspruch 8 oder 9, **dadurch gekennzeichnet, dass** der Schneidaktivierungsschalter (60) und der Koagulationsaktivierungsschalter (59) ausschließlich alternativ aktivierbar ausgebildet sind.

11. Instrument nach einem der vorstehenden Ansprüche, **dadurch gekennzeichnet, dass** der Transformator (49) an die Koagulationsspannungsquelle (54) angeschlossen oder anschließbar ist.

12. Instrument nach einem der vorstehenden Ansprüche, **dadurch gekennzeichnet, dass** die Koagulationsspannungsquelle (54) außerhalb des Instruments (11) angeordnet ist.

## Claims

1. Instrument for coagulation and targeted dissection of biological tissue,
with a tool (17) which has at least one first coagulation electrode (22) and one second coagulation electrode (29) that are movable relative to one another in order to hold tissue between them, and which has at least one cutting electrode (36) for cutting through tissue held between the least one first coagulation electrode (22) and the at least one second coagulation electrode (29),
with an electrical circuit (44) which is connected to the coagulation electrodes (22, 29) and to the cutting electrode (36) and contains or can be connected to a cutting voltage source (55) and a coagulation voltage source (54), wherein the cutting voltage source (55) is formed by a transformer (49) which is arranged in the instrument (11) and is or can be connected on the input side to the HF output voltage source (47), and wherein the HF output voltage source (47) serves to supply the coagulation voltage source (54),
wherein the circuit (44) has three output ports (M, K and S), of which the one output port (M) is a ground port connected to the at least one second coagulation electrode (29), the one further output port (K) is a coagulation port connected to the at least one first coagulation electrode (22), and the other further output port (S) is a port connected to the at least one cutting electrode (36) for a cutting voltage of the cutting voltage source (55),
wherein the electrical circuit (44) has a power switch (58) which is arranged in a current path between the cutting voltage source (55) and the cutting electrode (36) and is configured for optional isolation of the cutting electrode (36) from the cutting voltage source (55) or for optional connection of the cutting electrode (36) to the cutting voltage source (55), and an activation switch (62) which is configured for optional activation of the coagulation voltage source (54), the cutting voltage source (55) or the HF output voltage source (47),
wherein the activation switch (62) and the power switch (58) are controlled in series by a common actuation element (61).

2. Instrument according to claim 1, **characterised in that** on the input side, the electrical circuit (44) has three ports (45, 46, 48), wherein two ports (45, 46) are configured for connection to the HF output voltage source (47) in order to provide electrical energy for operation of the instrument (11), and wherein a third port (48) is configured for activation or deactivation of the HF output voltage source (47).

3. Instrument according to claim 1 or 2, **characterised in that** the power switch (58) and the activation switch (62) are connected to the actuation element (61) such that when the actuation element (61) is actuated, the power switch (58) closes before the activation switch (62) closes, and that when the actuation element (61) is de-actuated, the activation switch (62) opens before the power switch (58) opens.

4. Instrument according to any of the preceding claims, **characterised in that** the power switch (58) in rest state does not establish an electrical connection between the cutting voltage source (55) and the cutting electrode (36), and in actuated state connects the cutting electrode (36) to the cutting voltage source (55).

5. Instrument according to claim 4, **characterised in that** in rest state, the power switch (58) connects the cutting electrode (36) to the coagulation voltage source (54) or to a reduced voltage source (57) or is switched potential-free.

6. Instrument going to claim 5, **characterised in that** the reduced voltage source (57) is a transformer (49) which is connected to the coagulation voltage source (54) on the primary side.

7. Instrument according to claim 4, **characterised in that** in rest state, the power switch (58) connects the cutting electrode (36) to a current-limiting means (C3).

8. Instrument according to any of the preceding claims, **characterised in that** the instrument (11) has a coagulation activation switch (59) which is configured for activating the HF output voltage source (47).

9. Instrument according to any of the preceding claims, **characterised in that** the instrument (11) has a cutting activation switch (60) which is configured to activate the HF output voltage source (47).

10. Instrument according to claim 8 or 9, **characterised in that** the cutting activation switch (60) and the coagulation activation switch (59) are configured so as to be activatable exclusively alternatively.

11. Instrument according to any of the preceding claims, **characterised in that** the transformer (49) is or can be connected to the coagulation voltage source (54).

12. Instrument according to any of the preceding claims, **characterised in that** the coagulation voltage source (54) is arranged outside the instrument (11).

## Revendications

1. Instrument de coagulation et, si nécessaire, de dissection de tissus biologiques,
comprenant un outil (17) qui présente au moins une première électrode de coagulation (22) et une deuxième électrode de coagulation (29), qui peuvent être déplacées l'une par rapport à l'autre afin de saisir du tissu entre elles, et qui présente au moins une électrode de coupe (36) destinée à sectionner le tissu saisi entre la première électrode de coagulation (22), au nombre d'au moins une, et la deuxième électrode de coagulation (29), au nombre d'au moins une,
comprenant un circuit électrique (44) qui est raccordé aux électrodes de coagulation (22, 29) et à l'électrode de coupe (36) et comporte une source de tension de coupe (55) ainsi qu'une source de tension de coagulation (54) ou peut être raccordé à des sources de ce type, la source de tension de coupe (55) étant constituée d'un transformateur (49) qui est disposé dans l'instrument (11) et qui peut être raccordé ou qui est raccordé, côté entrée, à la source de tension de sortie HF (47), et la source de tension de sortie HF (47) servant à l'alimentation de la source de tension de coagulation (54),
le circuit (44) présentant trois bornes de sortie (M, K et S) parmi lesquelles la borne de sortie (M) est une borne de mise à la masse reliée à la deuxième électrode de coagulation (29), au nombre d'au moins une, l'autre borne de sortie (K) est une borne de coagulation reliée à la première électrode de coagulation (22), au nombre d'au moins une, et l'autre borne de sortie (S) est une borne reliée à l'électrode de coupe (36), au nombre d'au moins une, pour une tension de coupe de la source de tension de coupe (55),
le circuit électrique (44) présentant un disjoncteur (58), qui est placé dans un chemin de courant entre la source de tension de coupe (55) et l'électrode de coupe (36) et est conçu pour séparer, au choix, l'électrode de coupe (36) de la source de tension de coupe (55), ou pour relier, au choix, l'électrode de coupe (36) à la source de tension de coupe (55), et présentant un interrupteur d'activation (62) qui est conçu pour activer, au choix, la source de tension de coagulation (54), la source de tension de coupe (55) ou la source de tension de sortie HF (47), l'interrupteur d'activation (62) et le disjoncteur (58) étant commandés en série par un élément d'actionnement (61) commun.

2. Instrument selon la revendication 1, **caractérisé en ce que** le circuit électrique (44) présente trois bornes (45, 46, 48) côté entrée, deux bornes (45, 46) étant conçues pour le raccordement à la source de tension de sortie HF (47), en vue de fournir de l'énergie électrique pour le fonctionnement de l'instrument (11), et une troisième borne (48) étant conçue pour l'activation ou la désactivation de la source de tension de sortie HF (47).

3. Instrument selon la revendication 1 ou 2, **caractérisé en ce que** le disjoncteur (58) et l'interrupteur d'activation (62) sont reliés à l'élément d'actionnement (61) de manière à ce que lors d'une activation de l'élément d'actionnement (61), le disjoncteur (58) se ferme avant que l'interrupteur d'activation (62) ne se ferme, et de manière à ce que lors d'une libération de l'élément d'actionnement (61), l'interrupteur d'activation (62) s'ouvre avant que le disjoncteur (58) ne s'ouvre.

4. Instrument selon l'une des revendications précédentes, **caractérisé en ce que**, à l'état de repos, le disjoncteur (58) n'établit pas de liaison électrique entre la source de tension de coupe (55) et l'électrode de coupe (36) et, à l'état activé, relie l'électrode de coupe (36) à la source de tension de coupe (55).

5. Instrument selon la revendication 4, **caractérisé en ce que**, à l'état de repos, le disjoncteur (58) relie l'électrode de coupe (36) à la source de tension de coagulation (54) ou à une source de tension réduite (57) ou est commuté pour être libre de potentiel.

6. Instrument selon la revendication 5, **caractérisé en ce que** la source de tension réduite (57) est un transformateur (49) raccordé côté primaire à la source de tension de coagulation (54).

7. Instrument selon la revendication 4, **caractérisé en ce que**, à l'état de repos, le disjoncteur (58) relie l'électrode de coupe (36) à un moyen de limitation de courant (C3).

8. Instrument selon l'une des revendications précédentes, **caractérisé en ce que** l'instrument (11) présente un interrupteur d'activation de coagulation (59) qui est conçu pour l'activation de la source de tension de sortie HF (47).

9. Instrument selon l'une des revendications précédentes, **caractérisé en ce que** l'instrument (11) présente un interrupteur d'activation de coupe (60) qui est conçu pour l'activation de la source de tension de sortie HF (47).

10. Instrument selon la revendication 8 ou 9, **caractérisé en ce que** l'interrupteur d'activation de coupe (60) et l'interrupteur d'activation de coagulation (59) sont réalisés de manière à pouvoir être activés exclusivement de façon alternative.

11. Instrument selon l'une des revendications précédentes, **caractérisé en ce que** le transformateur (49) est raccordé ou peut être raccordé à la source de tension de coagulation (54).

12. Instrument selon l'une des revendications précédentes, **caractérisé en ce que** la source de tension de coagulation (54) est installée à l'extérieur de l'instrument (11).
